(19) Europäisches Patentamt
European Patent Office
Office européen des brevets

(11) Numéro de publication: **0 050 551**
**B1**

# (12) FASCICULE DE BREVET EUROPEEN

(45) Date de publication du fascicule du brevet: **18.04.84**

(51) Int. Cl.$^{3}$: **A 61 K 31/425,** C 07 D 277/82

(21) Numéro de dépôt: **81401564.0**

(22) Date de dépôt: **09.10.81**

(54) **Nouveau médicament à base d'amino-2 trifluorométhoxy-6 benzothiazole.**

(30) Priorité: **17.10.80 FR 8022218**

(43) Date de publication de la demande: **28.04.82 Bulletin 82/17**

(45) Mention de la délivrance du brevet: **18.04.84 Bulletin 84/16**

(84) Etats contractants désignés: **BE CH DE FR GB IT LI LU NL SE**

(56) Documents cités: **CHEMICAL ABSTRACTS, vol. 60, no. 1,6 janvier 1964, page 692, abrégé 692a-f, COLUMBUS, OHIO (US) L.M. YAGUPOL'SKII et al.: "Diazastyrenes containing fluorine"**

(73) Titulaire: **PHARMUKA LABORATOIRES, 35 Quai du Moulin de Cage, F-92231 Gennevilliers (FR)**

(72) Inventeur: **Mizoule, Jacques, Résidence Sisley 3, avenue de Verdun, F-92390 Villeneuve La Garenne (FR)**

(74) Mandataire: **Gaumont, Robert et al, RHONE-POULENC RECHERCHES Service Brevets Pharma 25, Quai Paul Doumer, F-92408 Courbevoie Cedex (FR)**

**EP 0 050 551 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

# Nouveau médicament à base d'amino-2 trifluorométhoxy-6-benzothiazole

La présente invention a pour objet un nouveau médicament, particulièrement utile comme anticonvulsivant, anxiolytique et hypnotique, qui contient, en tant qu'ingrédient actif, de l'amino-2 trifluoro-méthoxy-6 benzothiazole ou un sel de ce composé avec un acide pharmaceutiquement acceptable.

L'amino-2 trifluorométhoxy-6 benzothiazole, de formule:

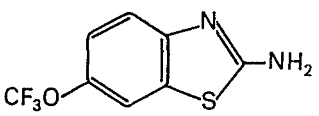

est un composé connu, qui peut être préparé par action du thiocyanate de potassium et du brome sur la trifluorométhoxy-4 aniline en milieu acide acétique (cf. Chem. Abstr. 60, 692 (1964) et L.M. Yagupol'skii, L.Z. Gandel'sman, Zh. Obshch. Khim. 33, 2301 (1963). Un mode opératoire utilisable pour cette préparation est le suivant:

A une solution de 84 g de trifluorométhoxy-4 aniline et 187 g de thiocyanate de potassium dans 500 ml d'acide acétique on ajoute goutte-à-goutte, en une heure, une solution de 77 g de brome dans 220 ml d'acide acétique. On agite à température ambiante pendant une nuit. Le mélange réactionnel est ensuite versé dans 2 l d'eau. On refroidit au bain de glace et neutralise par addition d'ammoniaque. L'insoluble est séparé par filtration, lavé à l'eau, puis recristallisé dans un mélange éthanol-eau (50-50). On obtient ainsi 77 g d'amino-2 trifluorométhoxy-6 benzothiazole, qui fond à 119°C.

Toutefois, si l'amino-2 trifluorométhoxy-6 benzothiazole est un composé connu, aucune propriété pharmacologique ou application thérapeutique n'a été signalée jusqu'ici pour ce composé.

Il a maintenant été trouvé, conformément à la présente invention, que ce composé possède des propriétés pharmacologiques remarquables qui permettent de l'utiliser comme ingrédient actif de médicaments.

## Propriétés pharmacologiques

1. Activité anticonvulsivante:

Malgré sa structure chimique très différente de celle des benzodiazépines, l'amino-2 trifluorométhoxy-6 benzothiazole agit comme celles-ci sur les mécanismes gabaergiques qui contrôlent un certain nombre de réactions psychomotrices d'origine centrale. Ceci est illustré par le fait que ce composé inhibe les convulsions provoquées chez l'animal par un inhibiteur de synthèse du GABA (acide $\gamma$-aminobutyrique) tel que l'isoniazide (INH), et cela à des doses 4 fois plus faibles que celles qui sont nécessaires pour inhiber les convulsions provoquées par le blocage des récepteurs centraux de la glycine par la strychnine. Ce n'est pas le cas d'autres agents anticonvulsivants non benzodiazépiniques tels le zoxazolamine dont l'action sur des convulsions provoquées par l'INH ou par la strychnine est pratiquement identique.

Ces effets ont été mis en évidence in vivo chez la souris et chez le rat.

Chez la souris, le produit est administré, en doses croissantes, par voie orale, à des lots de 10 souris mâles CD1 (Charles River) de 20–27 g, 90 minutes avant l'exposition à un électrochoc supramaximal (ESM), selon la méthode décrite par GM. Everett et R.K. Richards (J. Pharmacol. Exptl. Therap. 81, 402, 1944). Les résultats sont exprimés sous la forme d'une dose efficace 50% ($DE_{50}$), dose de produit qui protège 50% des animaux contre les convulsions provoquées par l'ESM.

Chez le rat mâle CD (Charles River) de 200–230 g, les convulsions sont provoquées par l'injection sous-cutanée soit de 500 mg/kg d'isoniazide (INH) soit de 1,5 mg/kg de strychnine sous forme de sulfate. Selon le protocole décrit par J.L. Costa et al. (Adv. Biochem. Pharmacol., 14, 113, 1975), le produit à tester est administré par voie intrapéritonéale 25 minutes après l'isoniazide et 5 minutes avant la strychnine. Le nombre d'animaux ayant présenté des convulsions est enregistré au cours d'une période d'observation de 60 minutes après l'injection de l'isoniazide et de 25 minutes après l'injection de la strychnine.

Dans chaque cas la $DE_{50}$ (dose de produit protégeant 50% des animaux contre les convulsions) est déterminée.

Les résultats obtenus sont rassemblés dans le tableau ci-après où figurent également, à titre comparatif, les résultats fournis par deux anticonvulsivants de référence (acide valproïque, phénobarbital).

| Produits | $DE_{50}$ (mg/kg) E.S.M. (souris vo) | I.N.H. (rat ip) | Strychnine (rat ip) |
|---|---|---|---|
| Amino-2 trifluoro-méthoxy-6 benzothiazole | 10 | 2,2 | 8,4 |
| Acide valproïque | 450 | 180 | inactif à 600 |
| Phénobarbital | 26 | 16 | 33 |

2. Activité anxiolytique

L'activité anxiolytique du produit a été mise en évidence chez la souris, en utilisant une méthode conflictuelle, dite test des 4 plaques, décrite par J.R. Boissier et al. (Europ. J. Pharmacol.-4, 145, 1968).

90 minutes après l'administration orale du produit, l'animal en expérience (souris mâle CD1 Charles River de 20–27 g) est placé dans une boîte dont le plancher est formé de quatre plaques métalliques séparées. Chaque fois que l'animal passe d'une plaque à une autre, on électrise l'ensemble des plaques. Rapidement l'animal associe déplacement et choc électrique et s'immobilise sur l'une des plaques. On détermine ainsi le nombre de traversées effectuées par la souris en 1 minute.

Les animaux sont répartis en groupes de 20 souris, le groupe contrôle ne recevant que le véhicule. Les résultats obtenus pour les groupes traités sont exprimé par un pourcentage d'augmentation du nombre de passages par rapport au groupe contrôle et sont rassemblés dans le tableau ci-dessous:

| Dose d'amino-2 trifluoro-méthoxy-6 benzothiazole en mg/kg, par voie orale | % d'augmentation du nombre de passages |
|---|---|
| 5 | + 50 |
| 10 | + 61 |
| 20 | +100 |
| 25 | +162 |
| 30 | +215 |

Propriétés toxicologiques

Les toxicités aiguës de l'amino-2 trifluorométhoxy-6 benzothiazole ont été déterminées chez la souris mâle $CD_1$ (Charles River) par les voies intrapéritonéale et orale. Les $DL_{50}$ ont été calculées, après 3 jours d'observation, par la méthode cumulative de J.J. Reed et H. Muench (Amer. J. Hyg., 27, 493, 1938). Les $DL_{50}$ obtenues sont rassemblées dans le tableau suivant:

| Voie d'administration | $DL_{50}$ mg/kg |
|---|---|
| Intrapéritonéale | 46 |
| Orale | 67 |

Utilisation therapeutique

Le médicament selon l'invention, qui contient de l'amino-2 trifluorométhoxy-6 benzothiazole ou un sel de ce composé avec un acide pharmaceutiquement acceptable associé à un véhicule pharmaceutiquement acceptable, peut être utilisé en thérapeutique humaine comme anticonvulsivant, anxiolytique et hypnotique. Il peut se présenter sous toutes les formes en usage dans le domaine des médicaments, telles que comprimés, capsules, gélules, suppositoires, solutions ou suspensions ingérables ou injectables, etc.

La posologie dépend des effets recherchés et de la voie d'administration utilisée. Par exemple, par voie orale, elle peut être comprise entre 5 et 250 mg de substance active par jour, avec des doses unitaires de 1 à 50 mg de substance active.

Les formules données ci-dessous pour le médicament selon l'invention ne sont données qu'à titre d'exemples et ne sont pas limitatives.

1. Formule «comprimés»:

a) Composition

| | Formule A | Formule B | Formule C |
|---|---|---|---|
| Amino-2 trifluorométhoxy-6 benzothiazole | 10 mg | 25 mg | 50 mg |
| Cellulose microcristalline | 20 mg | 75 mg | 75 mg |
| Mannitol | 50 mg | 41 mg | 41 mg |
| Polyvinyl pyrrolidone | 4 mg | 10 mg | 10 mg |
| Caséine méthylée | 10 mg | 0 | 0 |
| Carboxyméthylamidon (sel de sodium) | 0 | 25 mg | 25 mg |
| Silice colloïdale | 0 | 4 mg | 4 mg |
| Talc | 5 mg | 18 mg | 18 mg |
| Stéarate de magnésium | 1 mg | 2 mg | 2 mg |
| Total pour un comprimé | 100 mg | 200 mg | 225 mg |

b) Préparation

La matière active, après tamisage, est mélangée soigneusement dans un mélangeur planétaire avec le mannitol et la cellulose microcristalline. Le mouillage, destiné à l'obtention d'une masse granulable, est effectué à l'aide d'une solution méthanolique à 20% de poly(vinyl-1 pyrrolidone-2) et est complété, si nécessaire, à l'aide de méthanol. La masse humide est granulée sur un granulateur oscillant équipé d'une grille ayant une ouverture de maille de 3 mm. Le granulé est séché à 45°C dans une étuve ventilée, puis le granulé sec est calibré sur un tamis ayant une ouverture de maille de 1 mm. On ajoute ensuite le reste des adjuvants et mélange soigneusement, par exemple à l'aide d'un mélangeur planétaire. On comprime ensuite à l'aide d'une presse alternative ou rotative.

Les comprimés sont conditionnés en piluliers munis d'un capuchon en polyéthylène avec compensateur ou en blisters.

2. Formule «gélules»:

Les granulés obtenus comme indiqué précédemment peuvent être mis en gélules au lieu d'être comprimés. Par exemple les granulés correspondant à la formule B sont mis dans des gélules de taille no 2, chaque gélule contenant 200 mg de la composition.

Les gélules sont conditionnées en piluliers ou en blisters.

3. Formule «suppositoires»:

a) Composition:

| | |
|---|---|
| Amino-2 trifluorométhoxy-6 benzothiazole | 10 mg |
| Glycérides semi-synthétiques | q.s.p. 2 g |

b) Préparation:

On fond les glycérides semi-synthétiques par chauffage à 40°C et on incorpore par agitation la matière finement broyée (taille de particules inférieure à 100). On coule le mélange dans des alvéoles préformées en chlorure de polyvinyle et on laisse refroidir.

**Revendications**

1. Médicament utile comme anticonvulsivant, anxiolytique et hypnotique, contenant une substance active et un véhicule pharmaceutiquement acceptable, caractérisé en ce que la substance active est l'amino-2 trifluorométhoxy-6 benzothiazole ou un sel de ce composé avec un acide pharmaceutiquement acceptable.

2. Médicament selon la revendication 1 sous forme de dose unitaire contenant 1 à 50 mg de substance active.

**Claims**

1. Medicament, useful as anticonvulsant, anxiolytic and hypnotic, containing an active agent and a pharmaceutically acceptable carrier, characterised in that the active agent is 2-amino-6-trifluoromethoxybenzothiazole or a salt thereof with a pharmaceutically acceptable acid.

2. Medicament according to claim 1 in the form of a unitary dose containing 1 to 50 mg of active agent.

**Ansprüche**

1. Arzneimittel, das als Antikonvulsivum, als anxiolytisches Mittel und als Hypnotikum verwendbar ist und einen Wirkstoff und ein pharmazeutisch annehmbares Trägermaterial enthält, dadurch gekennzeichnet, dass der Wirkstoff ist 2-Amino-6-trifluormethoxy-benzothiazol oder ein Salz dieser Verbindung mit einer pharmazeutisch verträglichen Säure.

2. Arzneimittel nach Anspruch 1, in Form von einer Einzeldosis die 1 bis 50 mg von Wirkstoff enthält.